Europäisches Patentamt

European Patent Office

Office européen des brevets

(11) Publication number: 0 329 858
A1

## (12) EUROPEAN PATENT APPLICATION

(21) Application number: 88200324.7

(22) Date of filing: 23.02.88

(51) Int. Cl.4: A41B 13/02

(43) Date of publication of application:
30.08.89 Bulletin 89/35

(84) Designated Contracting States:
AT BE CH DE ES FR GB GR IT LI LU NL SE

(71) Applicant: Ancilla B.V.
Hoogveld 25 P.O. Box 19
NL-6590 AA Gennep(NL)

(72) Inventor: Berghmans, Johannes C.L.W.
Constantijnstraat 9
NL-5864 BO Meerlo(NL)

(74) Representative: Noz, Franciscus Xaverius, Ir.
et al
Algemeen Octrooibureau P.O. Box 645
NL-5600 AP Eindhoven(NL)

(54) Napkin.

(57) Napkin provided with a bottom layer impervious to moisture, with an upper layer (2) which is attached to the bottom layer (1) at its circumferential edge, and with a stuffing (8) of an absorbing material located between the two layers, whereby the upper layer is made of a material impervious to moisture, whilst a hole (3) is provided in the centre part of the upper layer.

FIG. 1

# NAPKIN

The invention relates to a napkin provided with a bottom layer impervious to moisture, with an upper layer which is attached to the bottom layer at its circumferential edge, and with a stuffing of an absorbing material located between the two layers.

In such napkins, known by themselves, also called incontinence napkins, the upper layer usually consists of a material pervious to moisture, so that the moisture can be absorbed by the stuffing consisting of an absorbing material. A disadvantage of such a design is, however, that if the moisture-absorbing material must absorb a lot of moisture, said moisture may escape near the boundary edge of the upper layer joining the bottom layer, which is experienced as particularly disadvantageous.

The purpose of the invention is to obtain a napkin of the above kind, wherein this disadvantage can be met.

According to the invention this can be achieved in that the upper layer is made of a material impervious to moisture, whilst a hole is provided in the centre part of the upper layer.

When using such an embodiment the moisture penetrating into the interior of the napkin via the hole in the upper layer can be absorbed by the moisture-absorbing material in the usual manner, whilst it is avoided that said moisture can escape again along the sides of the napkin.

If desired a protective layer, pervious to moisture, may furthermore be provided between the upper layer and the moisture-absorbing material.

According to a further aspect of the invention the moisture-absorbing material of the napkin is a mat of an absorbing material with flaps joining the sides of the centre part, said flaps being folded over the centre part whereby, in the longitudinal direction of the mat, one flap projects beyond the other flap at at least one side.

In this manner it will be possible to obtain, in a simple manner, a napkin wherein a thick layer of an absorbing material is located in the centre, whilst the thickness of said layer decreases towards the ends, so that while the moisture-absorbing capacity is great the napkin will still be comfortable to wear.

The invention will be further explained hereinafter with reference to an embodiment of a napkin according to the invention diagrammatically illustrated in the accompanying figures.

Fig 1 is a diagrammatic top view of an embodiment of a napkin according to the invention.

Fig 2 is a diagrammatic cross-section of fig 1 with the various parts of the napkin illustrated spaced from each other.

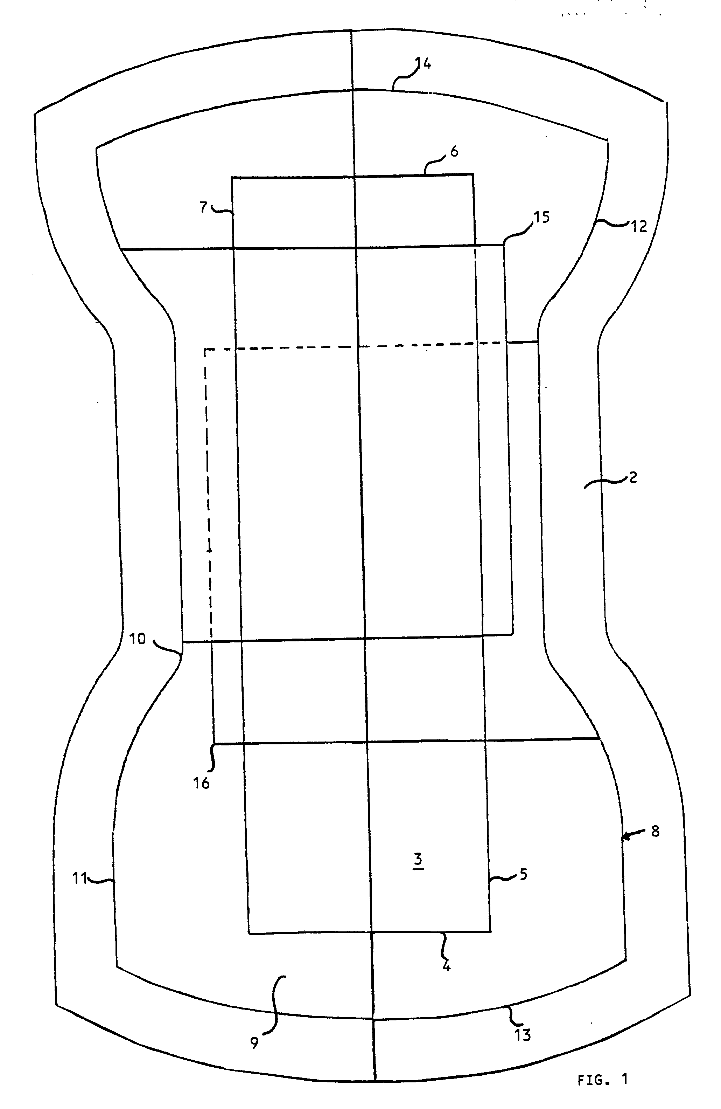

In the illustration of fig 1 it is assumed that the upper layer is transparent, so that parts located thereunder are also illustrated with full lines.

The napkin is provided with a bottom layer 1, impervious to moisture, and an upper layer 2 in which there is provided a centrally located elongated opening 3, which is bounded by boundary lines 4 - 7. Also the upper layer 2 is made of a material impervious to moisture.

Between the bottom layer 1 and the upper layer 2 there is located a stuffing 8 of an absorbing material. The stuffing 8 comprises a centre part 9 which comprises, as appears from fig 1, a centre portion 10 bounded by lines extending parallel to each other, said centre portion merging into parts 11 and 12 which become wider towards their ends, said parts 11 and 12 being bounded at their ends by curved boundary lines 13, 14 respectively. The edges of the bottom layer 1 and the upper layer 2 projecting beyond the stuffing 8 are attached together around the stuffing 8 in the usual manner. As appears from fig 1 the boundary lines of the outer layers 1 and 2 extend parallel to the boundary lines of the centre part 9 of the stuffing 8 thereby.

Near the centre of the centre part 9 of the stuffing 8 there are provided flaps 15 and 16 joining the sides of the centre part. Said flaps are folded down, such that the flap 16 abuts against the lower side of the centre part and the flap 15 abuts against the lower side of the flap 16. It is noted that for the sake of a clear illustration the flaps 15 and 16 are indicated by full lines in fig 1, in spite of the fact that said flaps are located under the parts 10 - 12, seen in fig 1. The design is thereby such that in the longitudinal direction of the napkin, seen in fig 1, the flap 15 projects beyond the the flap 16 near the upper end of the napkin and the flap 16 projects under the flap 15 near the bottom end of the napkin. So in the centre of the napkin the body 8 is built up of three layers lying on top of one another, whilst the thickness decreases towards the ends, first to two layers and then to one layer. In the centre of the napkin a large quantity of moisture-absorbing material is advantageously present, therefore, whilst the thickness of the moisture-absorbing stuffing body decreases towards the ends of the napkin, which makes wearing the napkin more pleasant. Also contributing towards the wearing comfort are the rounded ends 13 and 14 of the stuffing body and the corresponding shape of the boundary edges of the bottom layer 1 and the upper layer 2.

If desired it will also be possible to make the upper flap 15 a little shorter, in the longitudinal direction of the napkin, and to have the flap 16 project from the flap 15 at both ends.

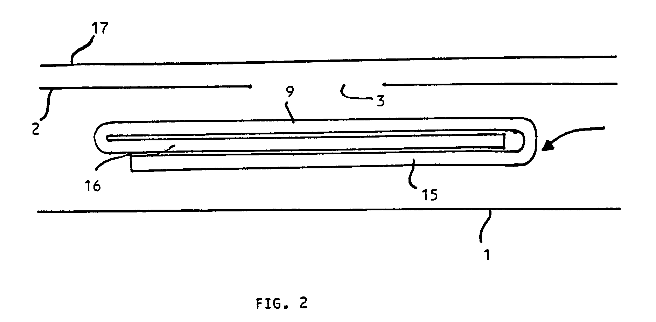

As is furthermore indicated in fig 2 a covering layer 17, pervious to moisture, may furthermore be provided over the upper layer 2 consisting of a material impervious to moisture, in order to further improve the wearing comfort, said covering layer having the same shape and dimensions as the layers 1 and 2 and being attached to said layers at its outer circumference.

## Claims

1. Napkin provided with a bottom layer impervious to moisture, with an upper layer which is attached to the bottom layer at its circumferential edge, and with a stuffing of an absorbing material located between the two layers, characterized in that the upper layer is made of a material impervious to moisture, whilst a hole is provided in the centre part of the upper layer.

2. Napkin according to claim 1, characterized in that an elongated hole, extending into the longitudinal direction of the napkin, is provided in the upper layer.

3. Napkin is particular according to claims 1 or 2, characterized in that between an upper layer and a bottom layer of the napkin there is provided a mat of an absorbing material with flaps joining the sides of the centre part of the mat, said flaps being folded down relatively to the centre part, whereby in the longitudinal direction of the mat one flap projects beyond the other flap, at least at one end.

4. Napkin according to claim 3, characterized in that in the longitudinal direction of the napkin one flap is located staggered relatively to the other flap.

5. Napkin according to any one of the preceding claims, characterized in that a covering layer of a material pervious to moisture is provided over the upper layer.

FIG. 1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | FR-A-1 359 810 (A. ASSEO) <br> * Whole document * | 1,5 | A 41 B 13/02 |
| X | FR-A-2 181 792 (PAPER CONVERTING MACHINE CO.) <br> * Page 2, lines 1-35; page 4, lines 9-23; claims 1,2; figures 1-3 * | 1,2,5 | |
| X | FR-A-2 299 824 (CONSORTIUM GENERAL · TEXTILE) <br> * Page 3, lines 13-40; page 6, lines 26-29; claims 1,4,5,7; figures 1,4 * | 1,2,5 | |
| A | FR-A-2 555 895 (WINKLER & DUNNEBIER MASCHINENFABRIK UND EISENGIESSEREI) <br> * Page 8, lines 1-20; figures 1-3 * | 3,4 | |
| E | NL-A-8 602 324 (ANCILLA) <br> * Whole document * | 1-5 | |

TECHNICAL FIELDS
SEARCHED (Int. Cl.4)

A 41 B
A 61 F

The present search report has been drawn up for all claims

| Place of search | Date of completion of the search | Examiner |
|---|---|---|
| THE HAGUE | 19-10-1988 | GARNIER F.M.A.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)